(19) Europäisches Patentamt
European Patent Office
Office européen des brevets

(11) Veröffentlichungsnummer: **0 421 267 A2**

(12) **EUROPÄISCHE PATENTANMELDUNG**

(21) Anmeldenummer: **90118530.6**

(22) Anmeldetag: **27.09.90**

(51) Int. Cl.[5]: **C07D 249/08**, A01N 43/653, A01N 43/88

(30) Priorität: **06.10.89 US 418225**

(43) Veröffentlichungstag der Anmeldung: **10.04.91 Patentblatt 91/15**

(84) Benannte Vertragsstaaten: **BE CH DE DK FR GB IT LI NL SE**

(71) Anmelder: **BASF Aktiengesellschaft**
**Carl-Bosch-Strasse 38**
**W-6700 Ludwigshafen(DE)**

(72) Erfinder: **Lauer, Manfred, Dr.**
**Im Zinkig 114**
**W-6700 Ludwigshafen(DE)**
Erfinder: **Zipplies, Matthias, Dr.**
**Kastanienweg 1**
**W-6945 Hirschberg(DE)**
Erfinder: **Sauter, Hubert, Dr.**
**Neckarpromenade 20**
**W-6800 Mannheim 1(DE)**
Erfinder: **Moore, Barbara Auxier**
**Route 4, Box 162**
**Pittsboro, N.C. 27312(US)**
Erfinder: **Carlson, Dale R., Dr.**
**107 King Street**
**Hillsborough, N.C. 27278(US)**
Erfinder: **Zorner, Paul Steffen, Dr.**
**19 Sanderling Court**
**Durham, N.C. 27713(US)**
Erfinder: **Westphalen, Karl-Otto,Dr.**
**Mausbergweg 58,**
**6720 Speyer,(DE)**
Erfinder: **Wuerzer, Bruno, Dr.**
**Ruedigerstrasse 13,**
**6701 Otterstadt,(DE)**

(54) **Synergistische Triazolverbindungen.**

(57) Triazolverbindungen I,

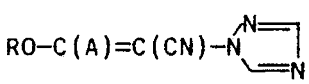

I

wobei die Reste folgende Bedeutung haben:
A
$C_7$-$C_{20}$-Alkyl;
ggf. substituiertes Phenyl, Naphthyl oder Pyridyl,
R
Wasserstoff;
ggf. substituiertes $C_1$-$C_6$-Alkyl, $C_3$-$C_6$-Alkenyl oder $C_3$-$C_6$-Alkinyl; $-COR^x$ oder $-SO_2R^x$, mit
$R^x$
Wasserstoff;
ggf. substituiertes $C_1$-$C_8$-Alkyl oder Phenyl
und deren umweltverträgliche Salze, sowie herbizide Mittel, enthaltend mindestens eine Triazolverbindung I, IA oder IB

EP 0 421 267 A2

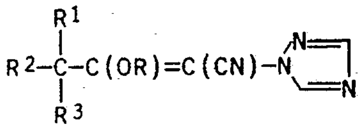

IA

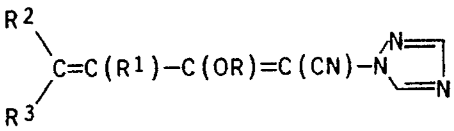

IB

wobei die Substituenten folgende Bedeutung haben:

$R^1$

Wasserstoff; Cyclopropyl;

ggf. substituiertes $C_1$-$C_6$-Alkyl, $C_2$-$C_6$-Alkenyl oder $C_2$-$C_6$-Alkinyl;

$C_1$-$C_4$-Alkoxy; $C_2$-$C_4$-Alkenyloxy oder $C_2$-$C_4$-Alkinyloxy;

$R^2$, $R^3$

Wasserstoff; Adamantyl;

$C_1$-$C_3$-Alkyl

oder gemeinsam mit dem C-Atom an das sie gebunden sind ggf. substituiertes $C_3$-$C_7$-Cycloalkyl, $C_6$-$C_{10}$-Bicycloalkyl oder $C_6$-$C_{10}$-Bicycloalkenyl

oder deren umweltverträgliche Salze sowie ein Benzothiadiazone.

## SYNERGISTISCHE TRIAZOLVERBINDUNGEN

Die vorliegende Erfindung betrifft Triazolverbindungen der allgemeinen Formel I

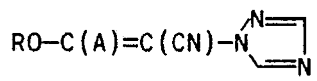

I

in der die Reste folgende Bedeutung haben:

A

$C_1$-$C_{20}$-Alkyl;

Phenyl, Naphthyl oder Pyridyl, wobei diese aromatischen Reste ein bis fünf Halogenatome, einen Phenyl- oder Phenoxyrest und/oder ein bis drei der folgenden Reste tragen können: Hydroxy, Nitro, Amino, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und/oder $C_1$-$C_4$-Halogenalkylthio;

R

Wasserstoff;

eine $C_1$-$C_6$-Alkylgruppe, eine $C_3$-$C_6$-Alkenylgruppe oder eine $C_3$-$C_6$-Alkinylgruppe, wobei diese Gruppen ein bis fünf Halogenatome und/oder einen der folgenden Reste tragen können: $C_1$-$C_4$-Alkoxy, $C_1$-$C_4$-Halogenalkoxy, $C_1$-$C_4$-Alkylthio, $C_1$-$C_4$-Halogenalkylthio, Phenyl;

einen Rest $COR^x$ oder einen Rest $SO_2R^x$, worin

$R^x$

Wasserstoff;

eine $C_1$-$C_8$-Alkylgruppe, welche ein bis drei Halogenatome tragen kann oder Phenyl, welches ein bis fünf Halogenatome und/oder ein bis drei der folgenden Reste tragen kann: Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, oder $C_1$-$C_4$-Halogenalkylthio bedeutet

und deren umweltverträgliche Salze, insbesondere solche, in denen A Phenyl bedeutet, welches ein- bis dreifach durch Halogen, $C_1$-$C_4$-Alkyl und/oder $C_1$-$C_2$-Halogenalkyl substituiert sein kann sowie herbizide Mittel, welche mindestens eine dieser Triazolverbindungen I oder mindestens eine Triazolverbindung der Formel IA oder IB,

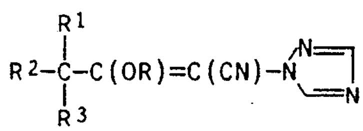

IA

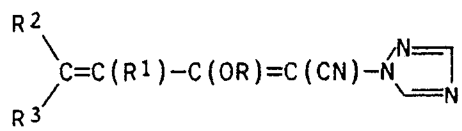

IB

enthalten, in der die Substituenten folgende Bedeutung haben:

$R^1$

Wasserstoff; eine Cyclopropylgruppe

eine $C_1$-$C_4$-Alkylgrupe, welche ein bis fünf Halogenatome und/oder ein bis drei der folgenden Gruppen tragen kann: Hydroxy, $C_1$-$C_4$-Alkoxy, $C_2$-$C_4$-Alkenyloxy oder $C_2$-$C_4$-Alkinyloxy;

eine $C_2$-$C_6$-Alkenylgruppe oder eine $C_2$-$C_6$-Alkinylgruppe, wobei diese Gruppen ein bis fünf Halogenatome tragen können;

eine $C_1$-$C_4$-Alkoxygrupe; eine $C_2$-$C_4$-Alkenyloxygruppe oder eine $C_2$-$C_4$-Alkinyloxygruppe;

$R^2$, $R^3$

Wasserstoff; Adamantyl;

eine $C_1$-$C_3$-Alkylgruppe

oder gemeinsam mit dem C-Atom an das sie gebunden sind eine $C_3$-$C_7$-Cycloalkylgruppe, eine $C_6$-$C_{10}$-Bicycloalkylgruppe oder eine $C_6$-$C_{10}$-Bicycloalkenylgruppe, wobei diese cyclischen Reste ein bis drei Halogenatome und/oder $C_1$-$C_3$-Alkylgruppen tragen können und

R

die in Anspruch 1 gegebene Bedeutung hat, oder deren umweltverträgliche Salze sowie mindestens einen herbiziden Wirkstoff aus der Gruppe der Benzothiadiazone der Formel II,

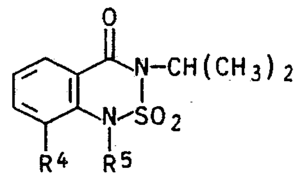

II

in der die Substituenten folgende Bedeutung haben:
$R^4$ Wasserstoff, eine $C_1$-$C_4$-Alkylgruppe, eine $C_1$-$C_4$-Alkoxygruppe oder ein Halogenatom;
$R^5$ Wasserstoff oder Cyano
sowie deren umweltverträgliche Salze
sowie hierfür übliche inerte Zusatzstoffe und Verfahren zur selektiven Bekämpfung von unerwünschten Pflanzenwuchs mit diesen herbiziden Mitteln.

Aus der EP-A-291 852 sind die vorstehend definierten Verbindungen IA und IB als Pflanzenwachstumsregulatoren bekannt.

Herbizide Wirkstoffe aus der Gruppe der Benzothiadiazone der Formel II

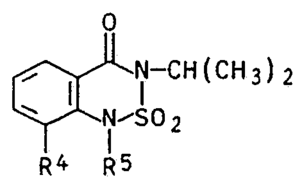

II

in der die Substituenten folgende Bedeutung haben:
$R^4$ Wasserstoff, eine $C_1$-$C_4$-Alkylgruppe, eine $C_1$-$C_4$-Alkoxygruppe oder ein Halogenatom;
$R^5$ Wasserstoff oder Cyano
sowie deren umwelt- und Kulturpflanzen verträgliche Salze dienen der Bekämpfung von unerwünschten Pflanzen (DE-A-15 42 836, US-A-4 158 559).

Der Erfindung lagen Verbindungen als Aufgabe zugrunde, welche die Wirkung der obengenannten Herbizide II gegen unerwünschte Pflanzen überadditiv steigern, ohne daß die Kulturpflanzenverträglichkeit verloren geht. Solche Verbindungen werden auch als Synergisten bezeichnet.

Entsprechend dieser Aufgabe wurden die eingangs definierten Triazolderivate I gefunden. Weiterhin wurden Verfahren zur gemeinsamen Anwendung dieser Verbindungen mit den bekannten Herbiziden II zur Bekämpfung unerwünschten Pflanzenwachstums gefunden. Außerdem wurde gefunden, daß die vorstehend definierten Triazolderivate IA und IB in Verbindungen mit den Herbiziden II ebenfalls synergistisch wirksam sind. Die Erfindung betrifft daher Mittel, welche die Verbindungen I, IA oder IB sowie Herbizide des Typs II enthalten sowie Verfahren zur Bekämpfung unerwünschten Pflanzenwuchses mit diesen Mitteln, wobei es unerheblich ist, ob der herbizide Wirkstoff und die synergistische Verbindung gemeinsam oder getrennt formuliert und ausgebracht werden bzw. bei getrennter Applikation, in welcher Reihenfolge herbizider Wirkstoff und Synergist ausgebracht werden.

Die erfindungsgemäßen Triazolderivate I sind auf verschiedenen Wegen zugänglich.

So erhält man die Verbindungen I, in denen R Wasserstoff bedeutet, (im weiteren als I′ bezeichnet), bevorzugt, indem man ein N-Cyanomethylentriazol III in an sich bekannter Weise (EP-A-291 852) in einem inerten aprotischen organischen Lösungsmittel in Gegenwart einer Base mit einem Carbonsäurehalogenid IV gemäß dem folgenden Schema umsetzt:

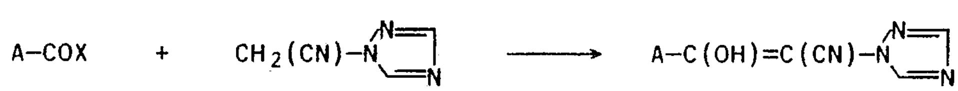

IV III I′ (R=H)

X in der Formel IV bedeutet dabei ein Halogenatom wie Fluor, Chlor und Brom, vorzugsweise Chlor und Brom oder der entsprechenden Anhydrid-Reste A-$CO_2$-.

Die Umsetzung kann kontinuierlich oder diskontinuierlich bei Normaldruck oder unter Drucken bis 30 bar, vorzugsweise bei Normaldruck oder 1 bis 10 bar, und Temperaturen von -50 °C bis 150 °C, vorzugsweise -20 °C bis 50 °C, durchgeführt werden.

Geeignete Lösungsmittel sind z. B. Ether wie Diethylether, Methyl-tert.-butylether, Tetrahydrofuran und Dioxan; chlorierte Kohlenwasserstoff wie Methylenchlorid, aromatische Kohlenwasserstoffe wie Toluol und Xylol; Ketone wie Aceton und Alkohole wie tert.-Butanol sowie Dimethylformamid oder Dimethylsulfoxid.

Als Basen kommen beispielsweise Hydroxide von Alkali- und Erdalkalimetallen wie Natriumhydroxid, Kaliumhydroxid und Calciumhydroxid, Alkoholate von Alkali- und Erdalkalimetallen wie Natriummethylat, Natriumethylat, Calciummethanolat oder Kalium-tert.-butylat; Alkali- oder Erdalkalimethallhydride wie Natriumhydrid, Kaliumhydrid oder Calciumhydrid, Alkali- oder Erdalkalicarbonate wie Natriumcarbonat, Kaliumcarbonat oder Calciumcarbonat; aliphatische Amine wie Dimethylamin, Triethylamin oder Diisopropylamin; heterocyclische Amine wie Piperidin, Piperazin oder Pyrrolidin; aromatische Amine wie Pyridin oder Pyrrol in Betracht.

Von diesen Basen werden im allgemeinen 1 bis 3 mol Äquivalent bezogen auf IV, vorzugsweise 1,9 bis 2,2 mol-Äquivalent, eingesetzt. Das molare Verhältnis der Edukte III zu IV beträgt im allgemeinen 1,5:1 bis 0,7:1, vorzugsweise 1,3:1 bis 0,8:1 und insbesondere 1,1:1 bis 0,9:1 mol-Äquivalent. Insbesondere erfolgt die Umsetzung in Tetrahydrofuran in Gegenwart von 1,9 bis 2,2 mol-Äquivalent Kalium-tert.-butylat als Base bei Temperaturen von -20 °C bis 30 °C unter Normaldruck.

Die Verbindungen der Formel I, in denen R nicht Wasserstoff bedeutet, (im folgenden als I″ bezeichnet), erhält man beispielsweise dadurch, daß man eine Triazolverbindung I′ in an sich bekannter Weise in einem aprotischen inerten organischen Lösungsmittel in Gegenwart einer Base mit einem elektrophilen Reagens V entsprechend der folgenden Reaktionsgleichung umsetzt.

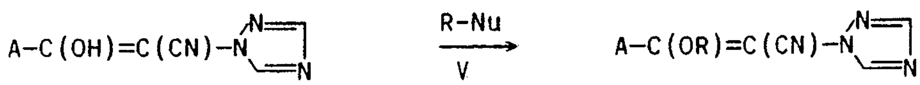


I′ (R=H) I″ (R≠H)

Nu in der Formel V bedeutet dabei eine nucleophile Abgangsgruppe wie Halogen, vorzugsweise Chlor oder Brom, oder sofern R eine Acylgruppe $-COR^x$ bedeutet, auch den entsprechenden Anhydridrest $R^xCO_2$.

Diese Umsetzung kann kontinuierlich oder diskontinuierlich, bei Normaldruck oder unter Drucken bis 30 bar, vorzugsweise bei Normaldruck oder einem Druck von 1 bis 10 bar, und Temperaturen von -20 °C bis 150 °C, vorzugsweise 20 °C bis 100 °C durchgeführt werden.

Geeignete aprotische Lösungsmittel sind sofern R eine Acylgruppe $-COR^x$ oder eine Sulfonylgruppe $-SO_2R^x$ bedeutet, besonders apolare Lösungsmittel wie die vorstehend genannten Ether, insbesondere Tetrahydrofuran. Als Basen eignen sich in diesem Fall organische Basen wie Alkoholate, insbesondere Natriummethylat, Natriumethylat und Kalium-tert.-butylat und tertiäre Amine wie insbesondere Triethylamin und Pyridin.

Sofern R ein Alkyl-, Alkenyl- oder Alkinylrest ist empfehlen sich für die Umsetzung aprotisch polare Lösungsmittel wie Cyclohexanon, Aceton und Acetonitril und die Gegenwart von anorganischen Basen wie insbesondere Kaliumcarbonat.

Diese Basen werden im allgemeinen in Konzentrationen von 0,5 bis 1,5 mol-Äquivalent, bezogen auf das Edukt I′, vorzugsweise 1 bis 1,1 mol-Äquivalent eingesetzt.

Das molare Verhältnis der Edukte I′ zu V beträgt im allgemeinen 2:1, vorzugsweise 1,5:1 und insbesondere 1:1 mol-Äquivalent.

Im Hinblick auf die bestimmungsgemäße Verwendung der Verbindungen I als Synergisten kommen als Substituenten beispielsweise folgende Reste in Betracht:

R

Wasserstoff;

Alkyl wie Methyl, Ethyl, Propyl, 1-Methylethyl, Butyl, 1-Methylpropyl, 2-Methylpropyl, 1,1-Dimethylpropyl, Pentyl, 2-Methylbutyl, 3-Methylbutyl, 2,2-Dimethylpropyl, Hexyl, 2-Methylpentyl, 3-Methylpentyl, 4-Methylpentyl, 2,2-Dimethylbutyl, 3,3-Dimethylbutyl, 2,3-Dimethylbutyl und 2-Ethylbutyl;

Alkenyl wie Allyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1,2-Dimethyl-2-propenyl, 1,1-Dimethyl-2-propenyl, 1-Methyl-3-butenyl, 1-Ethyl-2-propenyl, 2-Hexenyl, 1-Methyl-2-pentenyl, 1-Ethyl-2-butenyl und 2-Ethyl-2-butenyl, vorzugsweise Allyl oder Alkinyl wie 2-Propinyl, 2-Butinyl, 3-Butinyl, 1-Methyl-2-propinyl, 1-Methyl-2-butinyl, 1-Ethyl-2-propinyl, 2-Pentinyl, 3-Pentinyl, 4-Pentinyl und 1-Methyl-2-pentinyl, vorzugsweise 2-Propinyl;

wobei diese Gruppen ein bis fünf Halogenatome wie Fluor, Chlor, Brom und Iod, vorzugsweise Fluor und Chlor und/oder einen der folgenden Reste tragen können: Alkoxy wie Methoxy, Ethoxy, Propoxy, 1-Methylethoxy, Butoxy, 1-Methyloxy, 2-Methyloxy und 1,1-Dimethylethoxy; Halogenalkoxy wie Trifluormethoxy, Trichlormethoxy, Fluormethoxy, 2,2,2-Trifluorethoxy, 1,2,2-Trifluormethoxy und 1,1,2,2-Tetrafluorethoxy; Alkylthio wie Methylthio, Ethylthio, Propylthio, 2-Propylthio, Butylthio, 1-Methylpropylthio, 2-Methylpropylthio und 1,1-Dimethylethylthio; Halogenalkylthio wie vorzugsweise Trifluormethylthio und Trichlormethylthio, oder Phenyl;

ein Rest $COR^x$ oder ein Rest $SO_2R^x$, worin

$R)^x$

Alkyl wie bei R genannt, vorzugsweise Methyl,

welches ein bis drei Halogenatome wie insbesondere Fluor, Chlor und Brom tragen kann;

oder Phenyl bedeutet, welches ein bis fünf Halogenatome wie insbesondere Fluor und Chlor und/oder ein bis drei der folgenden Reste tragen kann:

Alkyl mit ein bis vier Kohlenstoffatome wie bei R genannt, insbesondere Methyl, Halogenalkyl wie Chlormethyl, Dichlormethyl, Trichlormethyl, 1,1-Dichlorethyl, 1-Chlorethyl, Fluormethyl, Difluormethyl, Trifluormethyl, 2-Fluoroethyl, 2,2-Difluormethyl und 2,2,2-Trifluorethyl;

Alkoxy wie vorstehend bei R genannt, insbesondere Methoxy, Halogenalkoxy wie vorstehend bei R genannt, insbesondere Trifluormethoxy; Alkylthio wie bei R genannt, insbesondere Methylthio; Halogenalkylthio wie insbesondere bei R genannt und

A

Alkyl mit 7 bis 20 Kohlenstoffatomen, welches vorzugsweise unverzweigt oder am Kettenende verzweigt ist wie Heptyl, 5-Methylhexyl, 4,4-Dimethylpentyl, Octyl, 6-Methylheptyl, 5,5-Dimethylhexyl, Nonyl, 7-Methyloctyl, 6,6-Dimethylheptyl, Decyl, 8-Methylnonyl, 7,7-Dimethyloctyl, Undecyl, 9-Methyldecyl, 8,8-Dimethylnonyl, Dodecyl, 10-Methylundecyl, 9,9-Dimethyldecyl, Tridecyl, 11-Methyldodecyl, 10, 10-Dimethylundecyl, Tetradecyl, 12-Methyltridecyl, 11,11-Dimethyldodecyl, Pentadecyl, 13-Methyltetradecyl, 12,12-Dimethyltridecyl, Hexadecyl, 14-Methylpentadecyl, 13,13-Dimethyltetradecyl, Heptedecyl, 15-Methylhexadecyl, 14,14-Dimethylpentadecyl, Octadecyl, 16-Methylheptadecyl, 15,15-Dimethylhexadecyl, Nonadecyl, 17-Methyloctadecyl, 16,16-Dimethylheptadeyl, Eicosyl, 18-Methylnonadecyl und 17,17-Dimethyloctadecyl, insbesondere unverzweigtes $C_7$-$C_{15}$-Alkyl;

Phenyl, Naphthyl oder Pyridyl, wobei diese aromatischen Reste ein bis fünf Halogenatome wie insbesondere Fluor und Chlor, einen Phenyl- oder Phenoxyrest und/oder ein bis drei der folgenden Reste tragen kann: Hydroxy, Nitro, Amino sowie Alkyl, Halogenalkyl, Alkoxy, Halogenalkoxy, Alkylthio und Halogenalkylthio wie im allgemeinen und im besonderen vorstehend bei $R^x$ genannt und/oder;

sowie deren umweltverträgliche Salze mit Alkalimetall- oder Erdalkalimetallionen wie Lithium, Natrium, Kalium, Magnesium und Calzium oder mit Ammoniumionen.

Einige der insbesondere bevorzugten Triazolverbindungen I sind in der folgenden Tabelle aufgeführt.

Tabelle

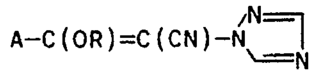 I

| A | R |
|---|---|
| 1-Naphthyl | H |
| 2-Naphthyl | H |
| p-Biphenyl | H |
| 3-Pyridyl | H |
| 2-Pyridyl | H |
| 4-Pyridyl | H |
| Phenyl | H |
| Phenyl | $CH_3$ |
| Phenyl | $C_2H_5$ |
| Phenyl | $n-C_3H_7$ |
| Phenyl | $n-C_4H_9$ |
| Phenyl | $n-C_5H_{11}$ |
| Phenyl | $n-C_6H_{13}$ |
| Phenyl | $COCH_3$ |
| Phenyl | 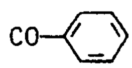 |
| Phenyl | $SO_2-CH_3$ |
| Phenyl | 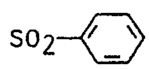 |
| Phenyl | 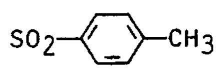 |
| Phenyl | 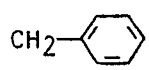 |
| Phenyl | $CH_2CH=CH_2$ |
| Phenyl | $Na^+$ |
| Phenyl | $K^+$ |
| Phenyl | $NH_4^+$ |

Tabelle (Fortsetzung)

| A | R |
|---|---|
| 2-F-phenyl | H |
| 2-F-phenyl | $CH_3$ |
| 2-F-phenyl | $C_2H_5$ |
| 2-F-phenyl | n-$C_3H_7$ |
| 2-F-phenyl | n-$C_4H_9$ |
| 2-F-phenyl | n-$C_5H_{11}$ |
| 2-F-phenyl | n-$C_6H_{13}$ |
| 2-F-phenyl | $COCH_3$ |
| 2-F-phenyl | CO 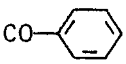 |
| 2-F-phenyl | $SO_2$-$CH_3$ |
| 2-F-phenyl | $SO_2$ 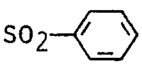 |
| 2-F-phenyl | $SO_2$ 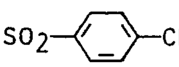 $CH_3$ |
| 2-F-phenyl | $CH_2$ 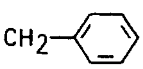 |
| 2-F-phenyl | $CH_2CH{=}CH_2$ |
| 2-F-phenyl | $Na^+$ |
| 2-F-phenyl | $K^+$ |
| 2-F-phenyl | $NH_4^+$ |
| 2-Cl-phenyl | H |
| 2-Cl-phenyl | $CH_3$ |
| 2-Cl-phenyl | $C_2H_5$ |
| 2-Cl-phenyl | n-$C_3H_7$ |
| 2-Cl-phenyl | n-$C_4H_9$ |
| 2-Cl-phenyl | n-$C_5H_{11}$ |
| 2-Cl-phenyl | n-$C_6H_{13}$ |
| 2-Cl-phenyl | $COCH_3$ |
| 2-Cl-phenyl | CO 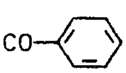 |
| 2-Cl-phenyl | $SO_2$-$CH_3$ |

Tabelle (Fortsetzung)

| A | R |
|---|---|
| 2-Cl-phenyl | $SO_2$— 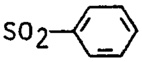 |
| 2-Cl-phenyl | $SO_2$— 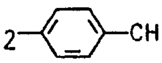 $CH_3$ |
| 2-Cl-phenyl | $CH_2$— 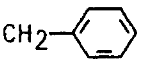 |
| 2-Cl-phenyl | $CH_2CH=CH_2$ |
| 2-Cl-phenyl | $Na^+$ |
| 2-Cl-phenyl | $K^+$ |
| 2-Cl-phenyl | $NH_4^+$ |
| 2,4-Cl,Cl-phenyl | H |
| 2,4-Cl,Cl-phenyl | $CH_3$ |
| 2,4-Cl,Cl-phenyl | $C_2H_5$ |
| 2,4-Cl,Cl-phenyl | $n-C_3H_7$ |
| 2,4-Cl,Cl-phenyl | $n-C_4H_9$ |
| 2,4-Cl,Cl-phenyl | $n-C_5H_{11}$ |
| 2,4-Cl,Cl-phenyl | $n-C_6H_{13}$ |
| 2,4-Cl,Cl-phenyl | $COCH_3$ |
| 2,4-Cl,Cl-phenyl | CO— 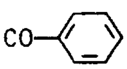 |
| 2,4-Cl,Cl-phenyl | $SO_2$— 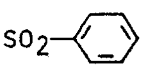 |
| 2,4-Cl,Cl-phenyl | $SO_2$— 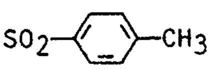 $CH_3$ |
| 2,4-Cl,Cl-phenyl | $CH_2$— 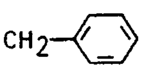 |
| 2,4-Cl,Cl-phenyl | $CH_2CH=CH_2$ |
| 2,4-Cl,Cl-phenyl | $Na^+$ |
| 2,4-Cl,Cl-phenyl | $K^+$ |
| 2,4-Cl,Cl-phenyl | $NH_4^+$ |

Tabelle (Fortsetzung)

| A | R |
|---|---|
| 2,6-Cl,Cl-phenyl | H |
| 2,6-Cl,Cl-phenyl | $CH_3$ |
| 2,6-Cl,Cl-phenyl | $C_2H_5$ |
| 2,6-Cl,Cl-phenyl | $n-C_3H_7$ |
| 2,6-Cl,Cl-phenyl | $n-C_4H_9$ |
| 2,6-Cl,Cl-phenyl | $n-C_5H_{11}$ |
| 2,6-Cl,Cl-phenyl | $n-C_6H_{12}$ |
| 2,6-Cl,Cl-phenyl | $COCH_3$ |
| 2,6-Cl,Cl-phenyl | CO 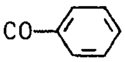 |
| 2,6-Cl,Cl-phenyl | $SO_2$ 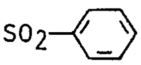 |
| 2,6-Cl,Cl-phenyl | $SO_2$ 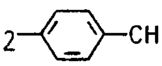 $CH_3$ |
| 2,6-Cl,Cl-phenyl | $CH_2$ 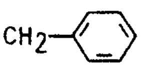 |
| 2,6-Cl,Cl-phenyl | $CH_2CH=CH_2$ |
| 2,6-Cl,Cl-phenyl | $Na^+$ |
| 2,6-Cl,Cl-phenyl | $K^+$ |
| 2,6-Cl,Cl-phenyl | $NH_4^+$ |
| 3,5-Cl,Cl-phenyl | H |
| 3,5-Cl,Cl-phenyl | $CH_3$ |
| 3,5-Cl,Cl-phenyl | $C_2H_5$ |
| 3,5-Cl,Cl-phenyl | $n-C_3H_7$ |
| 3,5-Cl,Cl-phenyl | $n-C_4H_9$ |
| 3,5-Cl,Cl-phenyl | $n-C_5H_{11}$ |
| 3,5-Cl,Cl-phenyl | $n-C_6H_{12}$ |
| 3,5-Cl,Cl-phenyl | $COCH_3$ |
| 3,5-Cl,Cl-phenyl | CO 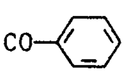 |
| 3,5-Cl,Cl-phenyl | $SO_2$ 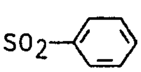 |

Tabelle (Fortsetzung)

| A | R |
|---|---|
| 3,5-Cl,Cl-phenyl | 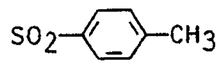 |
| 3,5-Cl,Cl-phenyl | 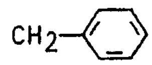 |
| 3,5-Cl,Cl-phenyl | $CH_2CH{=}CH_2$ |
| 3,5-Cl,Cl-phenyl | $Na^+$ |
| 3,5-Cl,Cl-phenyl | $K^+$ |
| 3,5-Cl,Cl-phenyl | $NH_4^+$ |
| 2,4,6-Trimethyl-phenyl | H |
| 2,4,6-Trimethyl-phenyl | $CH_3$ |
| 2,4,6-Trimethyl-phenyl | $C_2H_5$ |
| 2,4,6-Trimethyl-phenyl | $n-C_3H_7$ |
| 2,4,6-Trimethyl-phenyl | $n-C_4H_9$ |
| 2,4,6-Trimethyl-phenyl | $n-C_5H_{11}$ |
| 2,4,6-Trimethyl-phenyl | $n-C_6H_{12}$ |
| 2,4,6-Trimethyl-phenyl | $COCH_3$ |
| 2,4,6-Trimethyl-phenyl | 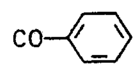 |
| 2,4,6-Trimethyl-phenyl | 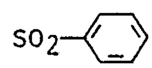 |
| 2,4,6-Trimethyl-phenyl | 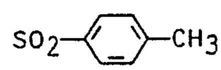 |
| 2,4,6-Trimethyl-phenyl | 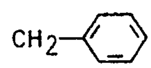 |
| 2,4,6-Trimethyl-phenyl | $CH_2CH{=}CH_2$ |
| 2,4,6-Trimethyl-phenyl | $Na^+$ |
| 2,4,6-Trimethyl-phenyl | $K^+$ |
| 2,4,6-Trimethyl-phenyl | $NH_4^+$ |
| 3-Cl-phenyl | H |
| 4-Cl-phenyl | H |
| 2-Br-phenyl | H |

Tabelle (Fortsetzung)

| A | R |
|---|---|
| 3-Br-phenyl | H |
| 4-Br-phenyl | H |
| 2-$CF_3$-phenyl | H |
| 2-$CF_3$-phenyl | n-$C_4H_9$ |
| 3-$CF_3$-phenyl | H |
| 4-$CF_3$-phenyl | H |
| 2-$CH_3$-phenyl | H |
| 2-$CH_3$-phenyl | Na |
| 2-$CH_3$-phenyl | n-$C_4H_9$ |
| 3-$CH_3$-phenyl | H |
| 4-$CH_3$-phenyl | H |
| 2-$CH_3O$-phenyl | H |
| 3-$CH_3O$-phenyl | H |
| 4-$CH_3O$-phenyl | H |
| 2,3-Cl,Cl-phenyl | H |
| 2,5-Cl,Cl-phenyl | H |
| 2-Cl,6-F-phenyl | H |
| 2-Cl,4-F-phenyl | H |
| 2-F,4-Cl-phenyl | H |
| 2,4-$CH_3$,$CH_3$-phenyl | H |
| 2,4-$CH_3$,$CH_3$-phenyl | n-$C_3H_7$ |
| 2,4-$CH_3$,$CH_3$-phenyl | n-$C_4H_9$ |
| 2,4-$CH_3$,$CH_3$-phenyl | n-$C_5H_{11}$ |
| 2,4-$CH_3$,$CH_3$-phenyl | n-$C_6H_{13}$ |
| 2,6-$CH_3$,$CH_3$-phenyl | H |
| $(CH_2)_9$-$CH_3$ | H |
| $(CH_2)_{14}CH_3$ | H |

Im Hinblick auf die bestimmungsgemäße Verwendung der Verbindungen IA und IB als Synergisten kommen als Substituenten beispielsweise folgende Reste in Betracht:

$R^1$

Wasserstoff; Cyclopropyl;

Alkyl wie vorstehend bei $R^x$ genannt, insbesondere Methyl welches ein bis drei Halogenatome wie insbesondere Fluor und Chlor und/oder ein bis drei der folgenden Gruppen tragen kann: Hydroxy, Alkoxy wie vorstehend bei $R^x$ genannt, insbesondere Methoxy und Ethoxy; Alkenyloxy wie Ethenyloxy, 1-Propenyloxy, 2-Propenyloxy, 1-Methyl-1-ethenyloxy, 1-Butenyloxy, 2-Butenyloxy, 3-Butenyloxy, 1-Methyl-1-propenyloxy, 1-Methyl-2-propenyloxy, 2-Methyl-1-propenyloxy, 2-Methyl-2-propenyloxy und 1-Ethyl-1-ethenyloxy, 1-Propinyloxy, 2-Propinyloxy, 1-Butinyloxy, 2-Butinyloxy, 3-Butinyloxy und 1-Methyl-2-propinyloxy;

Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butnyl, 2-Methyl-3-butenyl, 3-Methyl-3-butenyl, 1,1-Dimethyl-2-propenyl, 1,-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl3-pentenyl, 4-Methyl-3-pentenyl, 1-Mthyl-4-pentenyl,

2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, welches ein bis fünf Halogenatome wie insbesondere Fluor und Chlor tragen kann;

Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-3-butinyl, 2-Methyl-3-butinyl, 1-Methyl-2-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, welches ein bis fünf Halogenatome wie insbesondere Fluor und Chlor tragen kann;

Alkoxy wie vorstehend bei $R^x$ genannt, insbesondere Methoxy und Ethoxy;

Alkenyloxy wie im allgemeinen vorstehend genannt, oder

Alkinyloxy wie im allgemeinen vorstehend geannt, oder

$R^2$, $R^3$

Wasserstoff, Adamantyl, insbesondere 1-Adamantyl;

Alkyl mit ein bis drei Kohlenstoffatomen wie vorstehend genannt, insbesondere Methyl und Ethyl;

oder gemeinsam mit dem C-Atom an das sie gebunden sind Cycloalkyl wie Cyclopropyl, Cyclobutyl, Cyclopentyl, Cyclohexyl und Cycloheptyl, vorzugsweise Cyclohexyl, welches ein bis drei der folgenden Reste tragen kann: Halogen wie insbesondere Fluor und Chlor und/oder Alkyl wie insbesondere Methyl, Bicycloalkyl wie insbesondere Bicyclo[2.2.1]heptanyl (Norbornyl), Bicyclo[2.2.2]octanyl und Bicyclo[2.1.1]-hexanyl oder Bicycloalkenyl wie insbesondere Bicyclo[2.2.1]hepten-3-yl, wobei diese Bicyclen ein bis drei der folgenden Reste tragen können: Halogen wie insbesondere Chlor und Brom und/oder Alkyl wie insbesondere Methyl und Ethyl und

R

im allgemeinen und im besonderen wie vorstehend bei den Triazolderivaten I genannten Reste.

Spezielle, als Synergisten insbesondere bevorzugte Triazolverbindungen IA und IB sind in den folgenden Tabellen A und B aufgeführt.

Tabelle A:

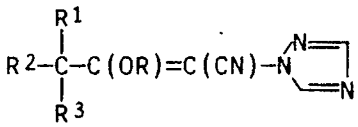

IA

| $R^1$ | $R^2$ | $R^3$ | R |
|---|---|---|---|
| H | H | H | H |
| $CH_3$ | H | H | H |
| $C_2H_5$ | H | H | H |
| $CH_2{=}CH$ | H | H | H |
| $CH{\equiv}C$ | H | H | H |
| $CH_3CH_2CH_2$ | H | H | H |
| $(CH_3)_2CH$ | H | H | H |
| Cyclopropyl | H | H | H |
| $CH_2{=}CHCH_2$ | H | H | H |
| $CH{\equiv}CCH_2$ | H | H | H |
| $CH_3CH_2CH_2CH_2$ | H | H | H |
| $(CH_3)_2CHCH_2$ | H | H | H |
| $CH_3CH(CH_3)CH_2$ | H | H | H |
| $(CH_3)_3C$ | H | H | H |
| $CH_2{=}CHCH_2CH_2$ | H | H | H |
| $CH{\equiv}CCH_2CH_2$ | H | H | H |
| $HOCH_2$ | H | H | H |
| $FCH_2$ | H | H | H |
| $ClCH_2$ | H | H | H |
| $CH_3O$ | H | H | H |
| $CH_3OCH_2$ | H | H | H |
| $CH_3CH_2OCH_2$ | H | H | H |
| $CH_2{=}CHCH_2OCH_2$ | H | H | H |
| $CH{\equiv}CCH_2OCH_2$ | H | H | H |
| $CH_3CH_2CH_2CH_2OCH_2$ | H | H | H |
| $CH_3$ | H | H | H |
| $C_2H_5$ | H | H | H |
| $CH_2{=}CH$ | H | H | H |
| $(CH_2)_5CH_3$ | H | H | H |
| $CH_3$ | $CH_3$ | H | H |
| $C_2H_5$ | $CH_3$ | H | H |
| $CH_2{=}CH$ | $CH_3$ | H | H |

Tabelle A (Fortsetzung)

| $R^1$ | $R^2$ | $R^3$ | R |
|---|---|---|---|
| $CH{\equiv}C$ | $CH_3$ | H | H |
| $CH_3CH_2CH_2$ | $CH_3$ | H | H |
| $(CH_3)_2CH$ | $CH_3$ | H | H |
| Cyclopropyl | $CH_3$ | H | H |
| $CH_2{=}CHCH_2$ | $CH_3$ | H | H |
| $CH{\equiv}CCH_2$ | $CH_3$ | H | H |
| $CH_3CH_2CH_2CH_2$ | $CH_3$ | H | H |
| $(CH_3)_2CHCH_2$ | $CH_3$ | H | H |
| $CH_3CH(CH_3)CH_2$ | $CH_3$ | H | H |
| $(CH_3)_3C$ | $CH_3$ | H | H |
| $CH_2{=}CHCH_2CH_2$ | $CH_3$ | H | H |
| $CH{\equiv}CCH_2CH_2$ | $CH_3$ | H | H |
| $HOCH_2$ | $CH_3$ | H | H |
| $FCH_2$ | $CH_3$ | H | H |
| $ClCH_2$ | $CH_3$ | H | H |
| $CH_3O$ | $CH_3$ | H | H |
| $CH_3OCH_2$ | $CH_3$ | H | H |
| $CH_3CH_2OCH_2$ | $CH_3$ | H | H |
| $CH_2{=}CHCH_2OCH_2$ | $CH_3$ | H | H |
| $CH{\equiv}CCH_2OCH_2$ | $CH_3$ | H | H |
| $CH_3CH_2CH_2CH_2OCH_2$ | $CH_3$ | H | H |
| $CH_3$ | $CH_3$ | $CH_3$ | H |
| $C_2H_5$ | $CH_3$ | $CH_3$ | H |
| $CH_2{=}CH$ | $CH_3$ | $CH_3$ | H |
| $CH{\equiv}C$ | $CH_3$ | $CH_3$ | H |
| $CH_3CH_2CH_2$ | $CH_3$ | $CH_3$ | H |
| $(CH_3)_2CH$ | $CH_3$ | $CH_3$ | H |
| Cyclopropyl | $CH_3$ | $CH_3$ | H |
| $CH_2{=}CHCH_2$ | $CH_3$ | $CH_3$ | H |
| $CH{\equiv}CCH_2$ | $CH_3$ | $CH_3$ | H |
| $CH_3CH_2CH_2CH_2$ | $CH_3$ | $CH_3$ | H |
| $(CH_3)_2CHCH_2$ | $CH_3$ | $CH_3$ | H |
| $CH_3CH(CH_3)CH_2$ | $CH_3$ | $CH_3$ | H |
| $(CH_3)_3C$ | $CH_3$ | $CH_3$ | H |
| $CH_2{=}CHCH_2CH_2$ | $CH_3$ | $CH_3$ | H |
| $CH{\equiv}CCH_2CH_2$ | $CH_3$ | $CH_3$ | H |
| $HOCH_2$ | $CH_3$ | $CH_3$ | H |

Tabelle A (Fortsetzung)

| $R^1$ | $R^2$ | $R^3$ | R |
|---|---|---|---|
| $FCH_2$ | $CH_3$ | $CH_3$ | H |
| $ClCH_2$ | $CH_3$ | $CH_3$ | H |
| $CH_3O$ | $CH_3$ | $CH_3$ | H |
| $CH_3OCH_2$ | $CH_3$ | $CH_3$ | H |
| $CH_3CH_2OCH_2$ | $CH_3$ | $CH_3$ | H |
| $CH_2=CHCH_2OCH_2$ | $CH_3$ | $CH_3$ | H |
| $CH\equiv CCH_2OCH_2$ | $CH_3$ | $CH_3$ | H |
| $CH_3CH_2CH_2CH_2OCH_2$ | $CH_3$ | $CH_3$ | H |
| $C_2H_5$ | $C_2H_5$ | H | H |
| $CH_2=CH$ | $C_2H_5$ | H | H |
| $CH\equiv C$ | $C_2H_5$ | H | H |
| $CH_3CH_2CH_2$ | $C_2H_5$ | H | H |
| $(CH_3)_2CH$ | $C_2H_5$ | H | H |
| $CH_2=CHCH_2$ | $C_2H_5$ | H | H |
| $CH\equiv CCH_2$ | $C_2H_5$ | H | H |
| $CH_3CH_2CH_2CH_2$ | $C_2H_5$ | H | H |
| $(CH_3)_2CHCH_2$ | $C_2H_5$ | H | H |
| $CH_3CH(CH_3)CH_2$ | $C_2H_5$ | H | H |
| $C_2H_5$ | $C_2H_5$ | $CH_3$ | H |
| $CH_2=CH$ | $C_2H_5$ | $CH_3$ | H |
| $CH\equiv C$ | $C_2H_5$ | $CH_3$ | H |
| $CH_3CH_2CH_2$ | $C_2H_5$ | $CH_3$ | H |
| $CH_3CH_2CH_2CH_2$ | $C_2H_5$ | $CH_3$ | H |
| $C_2H_5$ | $C_2H_5$ | $C_2H_5$ | H |
| $CH_3CH_2CH_2$ | $CH_2CH_3$ | $C_2H_5$ | H |
| $CH_3CH_2CH_2CH_2$ | $CH_2CH_3$ | $C_2H_5$ | H |
| $CH_3CH_2CH_2$ | n-$C_3H_7$ | H | H |
| $CH_3CH_2CH_2$ | n-$C_3H_7$ | $CH_3$ | H |
| $CH_3CH_2CH_2$ | n-$C_3H_7$ | $CH_2CH_3$ | H |
| $CH_3CH_2CH_2$ | n-$C_3H_7$ | n-$C_3H_7$ | H |
| H | $-CH_2CH_2-$ | | H |
| $CH_3$ | $-CH_2CH_2-$ | | H |
| H | $-CH_2CH_2CH_2-$ | | H |
| $CH_3$ | $-CH_2CH_2CH_2-$ | | H |
| H | $-CH_2CH_2CH_2CH_2-$ | | H |
| $CH_3$ | $-CH_2CH_2CH_2CH_2-$ | | H |
| H | $-CH_2CH_2CH_2CH_2CH_2-$ | | H |
| $CH_3-$ | $-CH_2CH_2CH_2CH_2CH_2-$ | | H |

Tabelle A (Fortsetzung)

| $R^1$ | $R^2$ | $R^3$ | R |
|---|---|---|---|
| H | Adamantyl | H | H |
| $CH_3$ | Adamantyl | H | H |
| $C_2H_5$ | $C_2H_5$ | H | COH |
| $C_2H_5$ | $C_2H_5$ | H | $COCH_3$ |
| $C_2H_5$ | $C_2H_5$ | H | $COC_2H_5$ |
| $C_2H_5$ | $C_2H_5$ | H | $COCH_2CH_3CH_3$ |
| $C_2H_5$ | $C_2H_5$ | H | $COCH_2CH_2CH_2CH_3$ |
| $C_2H_5$ | $C_2H_5$ | H | $COC(CH_3)_3$ |
| $C_2H_5$ | $C_2H_5$ | H | $COCH(CH_3)CH_2CH_3$ |
| $C_2H_5$ | $C_2H_5$ | H | $COCH(C_2H_5)_2$ |
| $C_2H_5$ | $C_2H_5$ | H | 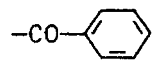 |
| $C_2H_5$ | $C_2H_5$ | H | 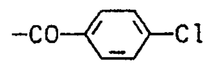 |
| $C_2H_5$ | $C_2H_5$ | H | 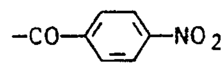 |
| $C_2H_5$ | $C_2H_5$ | H | 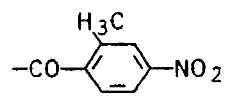 |
| $C_2H_5$ | $C_2H_5$ | H | 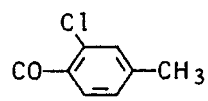 |
| $CH_3$ | $C_2H_5$ | H | $CO-CH_3$ |
| $CH_3$ | $CH_3$ | $CH_3$ | $SO_2CH_3$ |
| $CH_3$ | $CH_3$ | $CH_3$ | 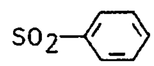 |
| $CH_3$ | $CH_3$ | $CH_3$ | 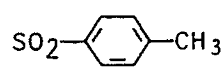 |
| $CH_3$ | $CH_3$ | $CH_3$ | 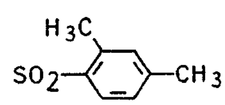 |
| H | $CH_3$ | $C_2H_5$ | $SO_2CH_3$ |

Tabelle A (Fortsetzung)

| $R^1$ | $R^2$ | $R^3$ | RR |
|---|---|---|---|
| H | $CH_3$ | $C_2H_5$ | 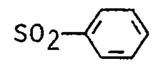 |
| H | $CH_3$ | $C_2H_5$ | 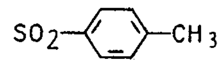 |
| H | $CH_3$ | $C_2H_5$ | 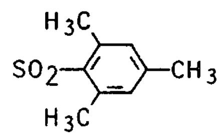 |
| H | $CH_3$ | $i-C_3H_7$ | $SO_2CH_3$ |
| H | $CH_3$ | $i-C_3H_7$ | 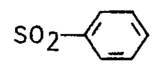 |
| H | $CH_3$ | $i-C_3H_7$ | 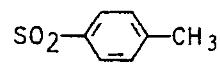 |
| H | $CH_3$ | $i-C_3H_7$ | 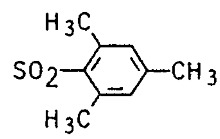 |
| H | $C_2H_5$ | $C_2H_5$ | $SO_2CH_3$ |
| H | $C_2H_5$ | $C_2H_5$ | 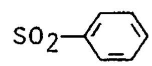 |
| H | $C_2H_5$ | $C_2H_5$ | 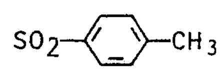 |
| H | $C_2H_5$ | $C_2H_5$ | 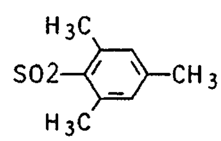 |
| H | $C_2H_5$ | $C_2H_5$ | 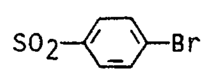 |
| H | $C_2H_5$ | $C_2H_5$ | 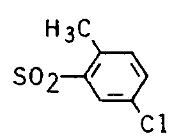 |

Tabelle A (Fortsetzung)

| $R^1$ | $R^2$ | $R^3$ | R |
|---|---|---|---|
| H | $C_2H_5$ | $C_2H_5$ | 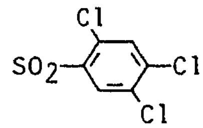 |
| H | $C_2H_5$ | $C_2H_5$ | 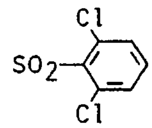 |
| H | 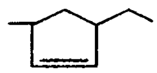 | | H |
| $CH_3$ | 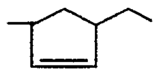 | | H |
| $C_2H_5$ | $C_2H_5$ | H | $(CH_3)_3N^{\oplus}CH_2CH_2Cl$ |
| $C_2H_5$ | $C_2H_5$ | H | $H_2N^{\oplus}(CH(CH_3)_2)_2$ |
| $C_2H_5$ | $C_2H_5$ | H | $H_2N^{\oplus}(CH_2CH_2OH)_2$ |
| $C_2H_5$ | $C_2H_5$ | H | $Na^{\oplus}$ |
| $CH_3$ | $CH_3$ | $CH_3$ | 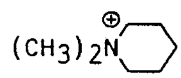 |
| $(CH_2)_5CH_3$ | H | H | $Na^{\oplus}$ |
| $C_2H_5$ | $CH_3$ | H | 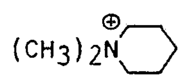 |
| $C_2H_5$ | $C_2H_5$ | H | 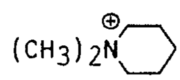 |
| $C(CH_3)_3$ | H | H | $Na^{\oplus}$ |
| $C_2H_5$ | $CH_3$ | H | $Na^{\oplus}$ |
| $(CH_2)_2CH_3$ | $CH_3$ | $CH_3$ | $Na^{\oplus}$ |
| $C_2H_5$ | H | H | $Na^{\oplus}$ |
| $CH(CH_3)_2$ | H | H | $Na^{\oplus}$ |

Tabelle B

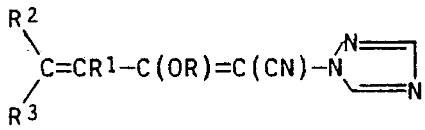

IB

| $R^1$ | $R^2$ | $R^3$ | R |
|---|---|---|---|
| H | H | H | H |
| H | $CH_3$ | H | H |
| H | $CH_3$ | $CH_3$ | H |
| H | $C_2H_5$ | H | H |
| H | $C_2H_5$ | $CH_3$ | H |
| H | $C_2H_5$ | $C_2H_5$ | H |
| $CH_3$ | H | H | H |
| $CH_3$ | $CH_3$ | H | H |
| $CH_3$ | $CH_3$ | $CH_3$ | H |
| $CH_3$ | $C_2H_5$ | H | H |
| $CH_3$ | $C_2H_5$ | $CH_3$ | H |
| $CH_3$ | $C_2H_5$ | $C_2H_5$ | H |
| $C_2H_5$ | H | H | H |
| $C_2H_5$ | $CH_3$ | H | H |
| $C_2H_5$ | $CH_3$ | $CH_3$ | H |
| $C_2H_5$ | $C_2H_5$ | H | H |
| $C_2H_5$ | $C_2H_5$ | $CH_3$ | H |
| $C_2H_5$ | $C_2H_5$ | $C_2H_5$ | H |
| H | $CH_3$ | $CH_3$ | $Na^{\oplus}$ |

Spezielle Beispiele für herbizide Benzothiadiazone II, deren Wirkung durch die synergistischen Triazolverbindungen I, IA und IB verbessert werden kann, sind in der folgenden Tabelle C aufgeführt:

Tabelle C

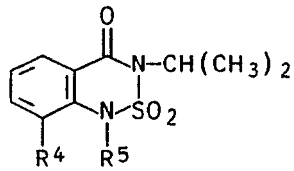

II

| Nr. | $R^4$ | $R^5$ | Literatur |
|---|---|---|---|
| II.001 | H | H | DE-A 15 42 836 |
| II.002 | Cl | H | DE-A 24 44 383 |
| II.003 | F | H | DE-A 24 44 383 |
| II.004 | $CH_3$ | H | DE-A 24 43 901 |
| II.005 | H | Na⊕ | DE-A 15 42 836 |
| II.006 | Cl | Na⊕ | DE-A 24 44 383 |
| II.007 | F | Na⊕ | DE-A 24 44 383 |
| II.008 | $CH_3$ | Na⊕ | DE-A 24 43 901 |
| II.009 | Cl | CN | DE-A 26 56 289 |
| II.010 | F | CN | DE-A 26 56 289 |
| II.011 | $CH_3$ | CN | DE-A 26 56 289 |
| II.012 | H | CN | DE-A 26 56 289 |

Für herbizide Benzothiadiazone der Formel II werden unterschiedliche Mengen einer synergistisch wirkenden Verbindung benötigt, wenn das Herbizid in verschiedenen Kulturen eingesetzt wird. Die Mengenverhältnisse sind in breiten Bereichen variabel. Sie sind ebenfalls abhängig von der Struktur der Benzothiadiazone der Formel II und der jeweiligen Zielkultur. Geeignete Anteilverhältnisse synergistisch wirkende : herbizider Wirkstoff Verbindung liegen bei 10:1 bis 0,01:1; vorzugsweise bei 6:1 bis 0,05:1, insbesondere bei 4:1 bis 0,1:1 Gew.-Teile.

Der herbizide Wirkstoff und die synergistisch wirkende Verbindung können gemeinsam oder getrennt nach dem Auflaufen auf die Blätter und Sprosse der Kulturpflanzen und der unerwünschten Pflanzen ausgebracht werden. Bevorzugt wird das synergistisch wirkende Mittel gleichzeitig mit dem herbiziden Wirkstoff ausgebracht. Auch eine getrennte Ausbringung, wobei der Synergist zuerst und anschließend der herbizide Wirkstoff auf das Feld gebracht werden, ist möglich. Herbizider Wirkstoff und Synergist können hierbei als Spritzmittel in suspendierbarer, emulgierbarer oder löslicher Form gemeinsam oder getrennt formuliert vorliegen.

Die erforderlichen Aufwandmengen an reiner Wirkstoffmenge, d.h. ohne Formulierungshilfsmittel ist abhängig von der Zusammensetzung des Pflanzenbestandes, vom Entwicklungsstadium der Pflanzen, von den klimatischen Verhältnissen am Einsatzort sowie von der Anwendungstechnik. Im allgemeinen betragen die Aufwandmengen 0,25 bis 5 kg, vorzugsweise 0,5 bis 2,5 kg Wirkstoff/ha.

Als Kulturen, in denen die erfindungsgemäßen herbiziden und pflanzenschützenden Mittel angewandt werden können, kommen im wesentlichen diejenigen in Betracht, in denen auch die Einzelwirkstoffe der Mischung eingesetzt werden können. Im Falle von Benzothiadiazonderivate der Formel II enthaltenden Mittel sind dies beispielsweise Getreide, Erdnüsse, Reis, Soja, Mais, Kultursorghum und Erbsen.

Von Bedeutung ist ferner die Ausbringungstechnik. Werden die neuen Mittel zum Zwecke der Bekämpfung unerwünschter Pflanzen bei Kulturpflanzen, die eine ungenügende Verträglichkeit aufweisen, eingesetzt, so wird nach speziellen Methoden die Ausbringung so gelenkt, daß die Blätter der Kulturpflanzen möglichst wenig mit den Mitteln in Berührung kommen, während die dazwischen oder darunter wachsenden unerwünschten Pflanzen oder die freie Bodenfläche von den Mitteln getroffen werden (Unterblattspritzung, post-directed, lay-by).

Die neuen herbiziden Mittel können neben dem Triazolderivat der Formel I, IA und/oder IB als Synergist und dem Herbizid aus der Gruppe der Benzothiadiazone II weitere herbizide und wachstumsregulierende Wirkstoffe andere inerte Zusätze enthalten, wobei der synergistische Effekt erhalten bleibt.

Neben der synergistischen Wirkung bei der gemeinsamen Anwendung mit Benzothiadiazinen der

Formel II können die Triazolderivate I, IA und IB auch bei folgenden Herbiziden als Synergisten eingesetzt werden (Handelsnamen in Klammern)

- 5-Amino-4-chlor-2-phenylpyridazin-3(2H)-on (Pyrazon)
- 4-Chlor-5-methylamino-2-(trifluormethylphenyl)-3(2H)-pyridazin-3(2H)-on (Monometfluorazon)
- 3-(3-Chlor-4-methylphenyl)-1,1-dimethylharnstoff (Chlortoluron)
- 3-(4-Bromphenyl)-1-methoxy-1-methylharnstoff (Metobromuron)
- 3-(4-Isopropylphenyl)-1,1-dimethylharnstoff (Isoproturon)
- 3(3,4-Dichlorphenyl)1-methoxy-1-methylharnstoff (Linuron)
- 3-(3,4-Dichlorphenyl)-1,1-dimethylharnstoff (Diuron)
- 3-(2-Benzothiazolyl)1,3-dimethylharnstoff (Methabenzthiazuron)
- 1,1-Dimethyl-3-(3-trifluormethylphenyl)-harnstoff (Fluometuron)
- 2-[3-(4-Methoxy-6-methyl-1,3,5-triazin-2-yl)aminocarbonylaminosulfonyl]benzsäure)methylester (Metsulfuron-methyl)
- 2-[3-(4,6-dimethoxypyrimidin-2-yl)aminocarbonylaminosulfonyl]benzoesäuremethylester (Bensulfuron-methyl)
- 2-[3-(4-Chlor-6-methoxypyrimidin-2-yl)aminocarbonylaminosulfonyl]benzoesäureethylester (Chlorimuron)
- 2-[3-(4-Methoxy-6-methyl-1,3,5-triazin-2-yl)-3-methylaminocarbonylaminosulfonyl]benzoesäuremethylester
- 3-(4,6-Dimethoxy-1,3,5-triazin-2-yl)-1-[2-(2-methoxyethoxy)phenylsulfonyl]harnstoff (Cinosulfuron)
- 2-[3-(4,6-bis(difluormethoxy)pyrimidin-2-yl)aminocarbonylaminosulfonyl]benzoesäuremethylester-(Primisulfuron)
- 2-(2-Chlorethoxy)N-[[(4-methoxy-6-methyl-1,3,5-triazin-2-yl)-aminocarbonyl]benzolsulfonsäureamid (Triasulfuron)
- 2-[[(4,6-Dimethoxypyrimidin-2-yl)aminocarbonyl]aminosulfonyl]-3-pyridincarbonsäure-N,N-dimethylamid
- S-(4-Chlorbenzyl)-N,N-diethylthiocarbamat (Benthiocarb)
- S-Benzyl-N,N-dipropylthiocarbamat (Prosulfocarb)
- S-Ethyl-N,N,-di-iso-butylthiocarbamat (Butylat)
- S-EthylN,N-di-n-propylthiocarbamat (EPTC)
- 3-(Methoxycarbonylamino)phenyl-N-(3-methylphenyl)carbamat (Phenmedipham)
- 3-(Ethoxycarbonylamino)phenyl-N-phenylcarbamat (Desmedipham)
- Isopropyl-N-(3-chlorphenyl)-carbamat (Chloropropham)
- 2,6-Dinitro-N,N-dipropyl-4-trifluormethylanilin (Trifluralin)
- 3,4-Dimethyl-2,6-Dinitro-N-1-ethylpropyl-anilin (Pendimenthalin)
- 4-Amino-3-methyl-6-phenyl-1,2,4-triazin-5(4H)-on (Metamitron)
- 4-Amino-6-tert.-butyl-3-methylthio-1,2,4-triazin-5(4H)-on (Metribuzin)

2-(2-chlor-4-ethylamino-1,3,5-triazin-yl-amino)-2-methylpropionitril
- (Cyanazin)

2-Chlor-4-ethylamino-6-iso-propylamino-1,3,5-triazin (Atrazin)
- 2-Chlor-4-ethylamino-6-tert.-butylamino-1,3,5-triazin
- (Terbutylazin)

3-Chlor-4-chromethyl-1-(3-trifluormethylphenyl)pyrrolidin-2-on
- (Fluorochloridin)

2-Chlor-6-nitro-3-phenoxyanilin (Aclonifen)
- 3,6-Dichlor-2-methoxybenzoesäure (Dicamba)
- 2,5-Dichlor-3-aminobenzoesäure (Amiben)
- 2,4-Dichlorphenoxyessigsäure (2,4-D)
- 2-(2,4-Dichlorphenoxy)propionsäure (Dichloprop)
- 2-(4-Chlor-2-methylphenoxy)propionsäure (Mecoprop)
- 2-[4-(2,4-Dichlorphenoxy)-phenoxy]propionsäuremethylester (Diclofop-methyl)
- 2[4-(6-Chlor-2-benzoxazolyloxy)phenoxy]propionsäureethylester (Fenoxaprop-ethyl)
- 2-[4-(6-Chlor-2-chinoxanyloxy)phenoxy]propionsäureethylester (Quizalafop-ethyl)
- 2-[4-(3-Chlor-5-trifluormethyl-2-pyridyloxy)phenoxy]propionsäuremethylester (Haloxyfop-methyl)
- 2-[4-(5-Trifluormethyl-2-pyridyloxy)phenoxy]propionsäurebutylester (Fluazifop-bentyl)

4-Amino-3,5-dichlor-6-fluor-2-pyridinyloxyessigsäure, dessen Salze und Alkylester, z.B. 1-Methylheptylester (Fluroxypyr)
- 7-Chlor-3-methyldinolin-8-carbonsäure (Quinmerac)
- 3,7-Dichlorchinolin-8-carbon-säure (Quinclorac)
- N-(2,4-Difluorphenyl)-2-(3-trifluormethylphenoxy)-3-pyridincarbon-säureamid (Diflufanican)
- exo-1-Methyl-4-(1-methylethyl)-2-(2-methylphenylmethoxy)-7-oxabicyclo(2.2.1)heptan (Cinmethlin)

- 2-(2-Chlorphenyl)methyl-4,4-dimethyl-3-is-oxxazolidinon (Clomazon)
- 5-Methylamino-2-phenyl-4-(3-trifluormethylphenyl)furan-3(2H)-on (Flurtamon)
- 2-[4,5-Dihydro-4-methyl-4-(1-methylethyl)-5-oxo-1H-imidazol-2-yl]-5-ethyl-3-pyridin-carbonsäure (Imazethapyr)
- 2-[4,5-Dihydro-4-methyl-4-(1-methylethyl)-5-oxo-1H-imidazol-2-yl)-3-chinolincarbonsäure (Imazaquin)
- 4-Chlor-2-oxobenzothiazolin-3-ylessigsäure (Benazolin)
- 2-Phenyl-3,1-benzoxazin-4-on
- 5-Fluor-2-phenyl-4H-3,1-benzoxazin-4-on (Fluorobentranil)
- 3′,4′-Dichlorpropionanilid (Propanil)
- 5-[2-Chlor-4-(trifluormethyl)phenoxyx]-2-nitrobenzoesäure-natriumsalz (Acifluorfen)
- 5-(2,4-Dichlorphenoxy)-2-nitrobenzoesäure-methylester (Bifenox)
- 5-(2-Chlor-4-trifluormethylphenoxy)-2-nitro-N-methansulfonylbenzoesäureamid (Fomesafen)
- 3,5-Dibrom-4-hydroxybenzonitril (Bromoxynil)
- 3,5-Dijod-4-hydroxybenzonitril (Ioxynil)

Außerdem ist es nützlich, die erfindungsgemäßen Mischungen auch noch mit weiteren Pflanzenschutzmitteln gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralstofflösungen, welche zur Behebung von Ernährungs- und Spurenelementmängeln eingesetzt werden.

Die erfindungsgemäßen Mittel bzw. bei getrennter Ausbringung die herbiziden Wirkstoffe oder der Synergist werden beispielsweise in Form von direkt versprühbaren Lösungen, Pulvern, Suspensionen, auch hochprozentigen wäßrigen, öligen oder sonstigen Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Die Formulierungen enthalten 0,1 bis 95 Gew.-%, vorzugsweise 0,5 bis 90 Gew.-% der Wirkstoffmischung. Sie können nach an sich bekannten Methoden durchgeführt werden.

Zur Herstellung von direkt versprühbaren Lösungen, Emulsionen, Pasten und Ölsdispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin-Sulfitablaugen und Methylcellulose in Betracht.

Pulver, Streu- und Stäubmittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Herstellungsbeispiele

Die in den nachstehenden Beispielen wiedergegebenen Vorschriften wurden unter entsprechender Abwandlung der Ausgangsverbindungen zur Gewinnung weiterer Verbindungen der Formeln I, IA und IB benutzt; die erhaltenen Verbindungen sind in den nachfolgenden Tabellen 1, 2 und 3 mit physikalischen Angaben aufgeführt.

Beispiel 1

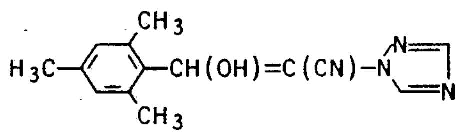

Eine Mischung aus 29,2 g (0,27 mol) Triazolylacetonitril, 49,3 g (0,27 mol) 2,4,6-Trimethylbenzoylchlorid und 250 ml Tetrahydrofuran wurde bei 0 °C portionsweise mit 60,6 g (0,54 mol) Kalium-tert.-butylat versetzt. Nach 12 Stunden Rühren bei 25 °C wurde das Lösungsmittel bei vermindertem Druck entfernt und der so erhaltenen Reaktionsrückstand in Wasser aufgenommen. Diese alkalische wäßrige Lösung des Produktes wurde extraktiv von Verunreinigungen befreit und anschließend angesäuert, wobei das Produkt als Feststoff anfiel.
Ausbeute: 44,4 g (65 %); Fp. > 200 °C
Wirkstoffbeispiel 1.021

Beispiel 2

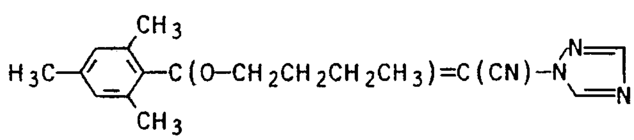

Eine Suspension aus 20,3 g (0,08 mol) des bei Beispiel 1 gewonnenen Produkts, 22,1 g (0,16 mol) Kaliumcarbonat und 100 ml Cyclopentanon wurde bei 25 °C mit 11,0 g (0,08 mol) Butylbromid versetzt und die so erhaltene Mischung wurde 8 Stunden bei 80 °C gerührt. Nach dem Abkühlen wurde vom Festkörper befreit und die so erhaltene Lösung bei vermindertem Druck eingeengt. Nach der üblichen Aufarbeitung wurde das Produkt chromatographisch gereinigt.
Ausbeute: 14,2 g (57 %); Fp. 60 - 70 °C
Wirkstoffbeispiel 1.022

Beispiel 3

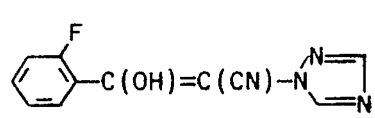

Aus 34,6 g (0,32 mol) Triazolylacetonitril und 50,7 g (0,32 mol) 2-Fluorbenzoylchlorid in 250 ml Tetrahydrofuran mit 71,8 g (0,64 mol) Kaliumtert.-butylat erhielt man analog Beispiel 1 62,7 g (74 %) des gewünschten Produktes.

Fp. 198 - 201 ° C;
Wirkstoffbeispiel 1.002

Beispiel 4

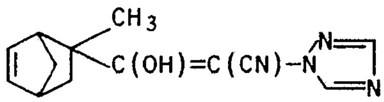

Aus 30,2 g (0,28 mol) Triazolylacetonitril und 47,8 g (0,28 mol) 1-Methyl(bicyclo[2.2.1]hept-2-en)-carbonsäurechlorid (Gemisch aus exo- und endo-Isomeren) in 250 ml Tetrahydrofuran mit 69,0 g (0,62 mol) Kalium-tert.-butylat erhielt man analog Beispiel 1 18 g (27 %) des gewünschten Produktes,
Fp. 85 - 88 ° C;
Wirkstoffbeispiel 2.041

Beispiel 5

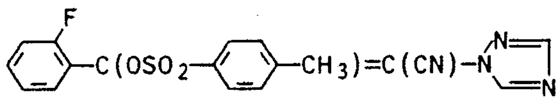

Eine Lösung aus 5,0 g (22 mmol) des bei Beispiel 3 erhaltenen Produktes in 150 ml Tetrahydrofuran wurde bei 25 ° C zunächst mit 0,1 g N,N-Dimethylaminopyridin und anschließend mit 4,5 g (24 mmol) p-Toluolsulfonsäurechlorid versetzt. Das so erhaltene Reaktionsgemisch wurde 3 Stunden bei 70 ° C gerührt und anschließend bei vermindertem Druck vom Lösungsmittel befreit. Nach üblicher Aufarbeitung fiel das Produkt des Feststoff an.
Ausbeute 5,8g (70%); Fp. 111 - 113 ° C;
Wirkstoffbeispiel 1.004

Beispiel 6

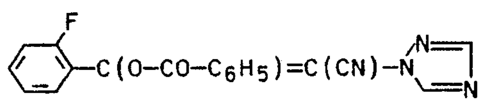

Analog zu Beispiel 5 erhielt man aus 5,0 g (22 mmol) des in Beispiel 3 hergestellten Produkts mit 3,4 g (24 mmol) Benzoylchlorid in 150 ml Tetrahydrofuran mit 0,1 g N,N-Dimethylamiopyridin 2,8 g (40 %) des gesuchten Produktes als Feststoff.
Fp. 116 - 118 ° C;
Wirkstoffbeispiel 1.003

Tabelle 1

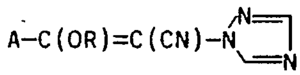

I

| Nr. | A | R | phys. Daten<br>Fp (°C); IR ($cm^{-1}$);<br>$^1H$-NMR (δ in ppm) |
|---|---|---|---|
| 1.001 | Phenyl | H | 141 - 142 |
| 1.002 | 2-F-phenyl | H | 198 - 201 |
| 1.003 | 2-F-phenyl | $CO-C_6H_5$ | 116 - 118 |
| 1.004 | 2-F-phenyl | 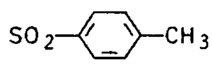 | 111 - 113 |
| 1.005 | 2-Cl-phenyl | H | 195 - 198 |
| 1.006 | 2-Cl-phenyl | $n-C_4H_9$ | 1630, 1503, 1433, 1321, 1172 |
| 1.007 | 2,4-Cl,Cl-phenyl | H | 210 - 212 |
| 1.008 | 2,4-Cl,Cl-phenyl | $n-C_4H_9$ | 81 - 85 |
| 1.009 | 2,4-Cl,Cl-phenyl | $n-C_6C_{13}$ | 64 - 66 |
| 1.010 | 2,4-Cl,Cl-phenyl | $CH_2-C_6H_5$ | 133 - 138 |
| 1.011 | 2,4-Cl,Cl-phenyl | $CH_2CH=CH_2$ | 90 - 92 |
| 1.012 | 2,6-Cl,Cl-phenyl | H | > 190 |
| 1.013 | 2,6-Cl,Cl-phenyl | $n-C_3H_7$ | 1363, 1502, 1431, 1311, 1174 |
| 1.014 | 2,6-Cl,Cl-phenyl | $n-C_4H_9$ | 89 - 101 |
| 1.015 | 2,6-Cl,Cl-phenyl | $n-C_5H_{11}$ | 1638, 1427, 1302 1230, 1148 |
| 1.016 | 2,6-Cl,Cl-phenyl | $n-C_6H_{12}$ | 2950, 2880, 2250, 1315, 1180 |

Tabelle 2

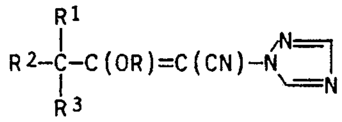

IA

| Nr. | $R^1$ | $R^2$ | $R^3$ | R | phys. Daten Fp (°C); IR ($cm^{-1}$); $^1H$-NMR (δ in ppm) |
|---|---|---|---|---|---|
| 2.001 | $CH_3$ | $CH_3$ | H | H | 78 - 80 |
| 2.002 | $C_2H_5$ | $CH_3$ | H | H | 87 |
| 2.003 | $CH_3CH_2CH_2$ | $CH_3$ | H | H | 2961, 2935, 1633, 1508 |
| 2.004 | $(CH_3)_2CH$ | $CH_3$ | H | H | 117 |
| 2.005 | $CH_3O$ | $CH_3$ | H | H | 117 - 118 |
| 2.006 | $CH_3$ | $CH_3$ | $CH_3$ | H | 2976, 1738, 1506, 1277 |
| 2.007 | $C_2H_5$ | $CH_3$ | $CH_3$ | H | 2973, 1736, 1505, 1278 |
| 2.008 | $CH_3CH_2CH_2$ | $CH_3$ | $CH_3$ | H | 2965, 1505, 1474, 1279 |
| 2.009 | $HOCH_2$ | $CH_3$ | $CH_3$ | H | [xHCl] 171 - 179 |
| 2.010 | $CH_3OCH_2$ | $CH_3$ | $CH_3$ | H | 2936, 1739, 1506, 1277 |
| 2.011 | $CH_3CH_2OCH_2$ | $CH_3$ | $CH_3$ | H | 1978, 1739, 1505, 1277, 1113 |
| 2.012 | $CH_3CH_2CH_2CH_2OCH_2$ | $CH_3$ | $CH_3$ | H | 1960, 1739, 1474, 1277 |
| 2.013 | $C_2H_5$ | $C_2H_5$ | H | H | 98 - 103 |
| 2.014 | $CH_3CH_2CH_2CH_2$ | $C_2H_5$ | H | H | 59 - 61 |
| 2.015 | $C_2H_5$ | $C_2H_5$ | $CH_3$ | H | 3112, 2969, 2207, 1520 |
| 2.016 | $CH_3CH_2CH_2$ | n-$C_3H_7$ | H | H | 88 - 91 |

Tabelle 2

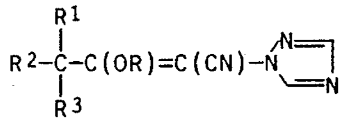

IA

| Nr. | $R^1$ | $R^2$ | $R^3$ | R | phys. Daten Fp (°C); IR ($cm^{-1}$); $^1H$-NMR (δ in ppm) |
|---|---|---|---|---|---|
| 2.001 | $CH_3$ | $CH_3$ | H | H | 78 - 80 |
| 2.002 | $C_2H_5$ | $CH_3$ | H | H | 87 |
| 2.003 | $CH_3CH_2CH_2$ | $CH_3$ | H | H | 2961, 2935, 1633, 1508 |
| 2.004 | $(CH_3)_2CH$ | $CH_3$ | H | H | 117 |
| 2.005 | $CH_3O$ | $CH_3$ | H | H | 117 - 118 |
| 2.006 | $CH_3$ | $CH_3$ | $CH_3$ | H | 2976, 1738, 1506, 1277 |
| 2.007 | $C_2H_5$ | $CH_3$ | $CH_3$ | H | 2973, 1736, 1505, 1278 |
| 2.008 | $CH_3CH_2CH_2$ | $CH_3$ | $CH_3$ | H | 2965, 1505, 1474, 1279 |
| 2.009 | $HOCH_2$ | $CH_3$ | $CH_3$ | H | [xHCl] 171 - 179 |
| 2.010 | $CH_3OCH_2$ | $CH_3$ | $CH_3$ | H | 2936, 1739, 1506, 1277 |
| 2.011 | $CH_3CH_2OCH_2$ | $CH_3$ | $CH_3$ | H | 1978, 1739, 1505, 1277, 1113 |
| 2.012 | $CH_3CH_2CH_2CH_2OCH_2$ | $CH_3$ | $CH_3$ | H | 1960, 1739, 1474, 1277 |
| 2.013 | $C_2H_5$ | $C_2H_5$ | H | H | 98 - 103 |
| 2.014 | $CH_3CH_2CH_2CH_2$ | $C_2H_5$ | H | H | 59 - 61 |
| 2.015 | $C_2H_5$ | $C_2H_5$ | $CH_3$ | H | 3112, 2969, 2207, 1520 |
| 2.016 | $CH_3CH_2CH_2$ | n-$C_3H_7$ | H | H | 88 - 91 |

Tabelle 2 (Fortsetzung)

| Nr. | $R^1$ | $R^2$ | $R^3$ | R | phys. Daten Fp (°C); IR ($cm^{-1}$); $^1H$-NMR (δ in ppm) |
|---|---|---|---|---|---|
| 2.017 | H | $-CH_2CH_2-$ | | H | 2209, 1592, 1508, 1277 |
| 2.018 | $CH_3$ | $-CH_2CH_2-$ | | H | 152 - 156 |
| 2.019 | H | $-CH_2CH_2CH_2-$ | | H | 2950, 1624, 1508, 1277 |
| 2.020 | H | $-CH_2CH_2CH_2CH_2-$ | | H | 81 - 82 |
| 2.021 | H | $-CH_2CH_2CH_2CH_2CH_2-$ | | H | 121 - 125 |
| 2.022 | $CH_3$ | $-CH_2CH_2CH_2CH_2CH_2-$ | | H | 2935, 1504, 1277, 1134 |
| 2.023 | H | 1-Adamantyl | H | H | 114 - 117 |
| 2.024 | $C_2H_5$ | $C_2H_5$ | H | $COCH_3$ | 2970, 1757, 1505, 1205 |
| 2.025 | $C_2H_5$ | $C_2H_5$ | H | $COC_2H_5$ | 2867, 1785, 1506, 1087 |
| 2.026 | $C_2H_5$ | $C_2H_5$ | H | $CO-C_6H_5$ | 2970, 1757, 1505, 1205 |
| 2.027 | $C_2H_5$ | $C_2H_5$ | H | 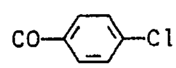 | 1758, 1593, 1204, 1089 |
| 2.028 | $C_2H_5$ | $C_2H_5$ | H | 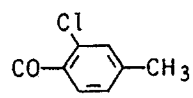 | 2967, 1770, 1585, 1201 |
| 2.029 | $CH_3$ | $C_2H_5$ | H | $COCH_3$ | 2970, 2937, 2217, 1633 |
| 2.030 | $CH_3$ | $CH_3$ | $CH_3$ | $SO_2CH_3$ | 1362, 1187, 1088, 795 |
| 2.031 | H | $CH_3$ | $C_2H_5$ | $SO_2CH_3$ | 60 - 65 |
| 2.032 | H | $CH_3$ | $C_2H_5$ | 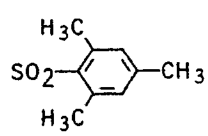 | 1371, 1176, 1210, 1053 |

Tabelle 2 (Fortsetzung)

| Nr. | $R^1$ | $R^2$ | $R^3$ | R | phys. Daten Fp (°C); IR ($cm^{-1}$); $^1$H-NMR (δ in ppm) |
|---|---|---|---|---|---|
| 2.033 | H | $CH_3$ | i-$C_3H_7$ | 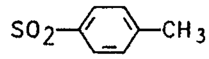 | 30 - 40 |
| 2.034 | H | $C_2H_5$ | $C_2H_5$ | $SO_2CH_3$ | 1506, 1372, 1210, 1177 |
| 2.035 | H | $C_2H_5$ | $C_2H_5$ | 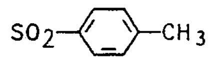 | 78 - 87 |
| 2.036 | H | $C_2H_5$ | $C_2H_5$ | 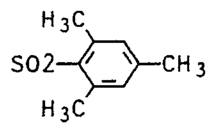 | 2968, 1054, 1370, 1175 |
| 2.037 | H | $C_2H_5$ | $C_2H_5$ | 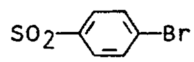 | 108 - 114 |
| 2.038 | H | $C_2H_5$ | $C_2H_5$ | 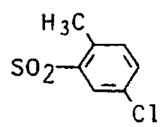 | 112 - 116 |
| 2.039 | H | $C_2H_5$ | $C_2H_5$ | 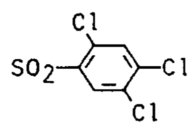 | 137 - 139 |
| 2.040 | H | $C_2H_5$ | $C_2H_5$ | 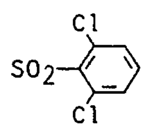 | 100 - 104 |
| 2.041 | $CH_3$ | 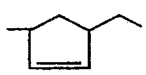 | | H | 85 - 88 |
| 2.042 | H | H | H | H | 155 |
| 2.043 | $CH_3$ | H | H | H | 134 - 136 |
| 2.044 | $CH_2CH_3$ | H | H | H | 108 |
| 2.045 | $CH(CH_3)_2$ | H | H | H | 126 |
| 2.046 | $C(CH_3)_3$ | H | H | H | 143 |
| 2.047 | $(CH_2)_5CH_3$ | H | H | H | 61 - 63 |
| 2.048 | $CH_2CH_3$ | $CH_2CH_3$ | H | $(CH_3)_3N^{\oplus}CH_2CH_2Cl$ | 8,50(s,1H); 7,85(s,1H) |

Tabelle 2 (Fortsetzung)

| Nr. | $R^1$ | $R^2$ | $R^3$ | R | phys. Daten Fp (°C); IR ($cm^{-1}$); $^1H$-NMR (δ in ppm) |
|---|---|---|---|---|---|
| 2.049 | $CH_2CH_3$ | $CH_2CH_3$ | H | $H_2N^{\oplus}(CH(CH_3)_2)_2$ | 2962, 2170, 1539 1275 |
| 2.050 | $CH_2CH_3$ | $CH_2CH_3$ | H | $H_2N^{\oplus}(CH_2CH_2OH)_2$ | 2961, 2175, 1537, 1457 |
| 2.051 | $CH_2CH_3$ | $CH_2CH_3$ | H | $Na^{\oplus}$ | 95 - 100 |
| 2.052 | $CH_3$ | $CH_3$ | $CH_3$ | 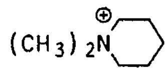 | 2951, 2151, 1533, 1480 |
| 2.053 | $(CH_2)_5CH_3$ | H | H | $Na^{\oplus}$ | 67 - 70 |
| 2.054 | $CH_2CH_3$ | $CH_3$ | H | 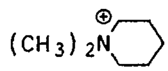 | 8,45(s,1H); 8,15(s,1H) |
| 2.055 | $CH_2CH_3$ | $CH_2CH_3$ | H | 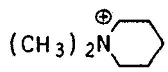 | 8,45(s,1H); 8,15(s,1H) |
| 2.056 | $C(CH_3)_3$ | H | H | $Na^{\oplus}$ | > 200 |
| 2.057 | $CH_2CH_3$ | $CH_3$ | H | $Na^{\oplus}$ | 130 - 140 |
| 2.058 | $(CH_2)_2CH_3$ | $CH_3$ | $CH_3$ | $Na^{\oplus}$ | 132 - 133 |
| 2.059 | $CH_2CH_3$ | H | H | $Na^{\oplus}$ | 114 - 121 |
| 2.060 | $CH(CH_3)_2$ | H | H | $Na^{\oplus}$ | > 200 |
| 2.061 | H | H | H | $Na^{\oplus}$ | 133 - 135 |
| 2.062 | H | $-CH_2CH_2-$ | | $Na^{\oplus}$ | 106 - 112 |
| 2.063 | H | $-(CH_2)_5$ | | $Na^{\oplus}$ | 112 - 125 |
| 2.064 | $CH_3$ | $CH_3$ | $n-C_3H_7$ | $Na^{\oplus}$ | 87 - 98 |
| 2.065 | $CH_3$ | $CH_3$ | $CH_3$ | 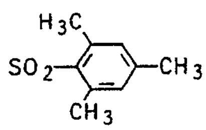 | 170 - 175 |
| 2.066 | H | $C_2H_5$ | $C_2H_5$ | 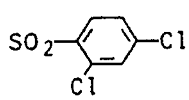 | 112 - 116 |

Tabelle 3

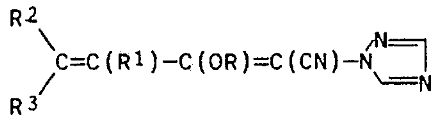

IB

| Nr. | $R^1$ | $R^2$ | $R^3$ | R | phys. Daten Fp (°C); IR ($cm^{-1}$); $^1H$-NMR (δ in ppm) |
|---|---|---|---|---|---|
| 3.001 | H | $CH_3$ | $CH_3$ | H | 118 |
| 3.002 | $CH_3$ | $CH_3$ | H | H | 64 - 71 |
| 3.003 | $C_2H_5$ | $CH_3$ | H | H | 81 - 88 |
| 3.004 | H | $CH_3$ | $CH_3$ | $Na^{\oplus}$ | > 200 |

Anwendungsbeispiele

Die synergistische Wirkung der Triazolverbindungen der Formeln I, IA und IB ließ sich durch Gewächshausversuche zeigen:

Zur Aufzucht der Testpflanzen dienten Plastikblumentöpfe mit 300 $cm^3$ Inhalt und lehmigem Sand mit etwa 3 % Humus als Substrat. Die Samen der Testpflanzen wurden nach Arten getrennt flach eingesät.

Bei Vorauflaufbehandlung wurden die aufbereiteten Mittel unmittelbar danach auf die Erdoberfläche aufgebracht. Die Wirkstoffe wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen. Danach wurden die Gefäße mit durchsichtigen Plastikhauben abgedeckt, bis die Pflanzen angewaschsen waren. Diese Abdeckung fördert ein gleichmäßiges Keimen der Testpflanzen, sofern dies nicht durch die Wirkstoffe beeinträchtigt wird.

Zum Zwecke der Nachauflaufbehandlung wurden die Testpflanzen je nach Wuchsform erst bis zu einer Wuchshöhe von 3 bis 15 cm angezüchtet und erst dann mit den in Wasser suspendierten oder emulgierten Wirkstoffen behandelt. Die Testpflanzen wurden dafür entweder direkt gesät und in den gleichen Gefäßen aufgezogen oder sie wurden als Keimpflanzen getrennt gezüchtet und einige Tage vor der Behandlung in die Versuchsgefäße verpflanzt. Die Aufwandmengen für die Nachauflaufbehandlung betrugen 0,125 und 1,5 kg Wirkstoff/ha. Eine Abdeckung unterblieb bei der Nachauflaufbehandlung.

Die Versuchsgefäße wurden im Gewächshaus aufgestellt, wobei für wärmeliebende Arten wärmere Bereiche (20 bis 35° C) und für solche gemäßigter Klimate 10 bis 25° C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen: Amaranthus retroflexus, Glycine max.

Beispielhaft wurden folgende Wirkstoffe bzw. entsprechende Wirkstoffgemische eingesetzt:

Als Vertreter der Benzothiadiazone II diente Beispiel II.005.

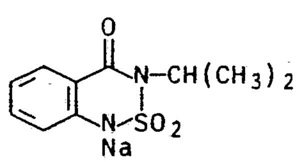

II.005

Der Wirkstoff wurde als wäßrige Lösung in einer Konzentration von 480 g/l formuliert.

Als Vertreter der synergistischen Triazolverbindungen I diente der Wirkstoff Nr. 1.002, als Beispiel für die Triazolverbindungen IA diente der Wirkstoff 2.041. Beide Triazolverbindungen wurden als 10 % Emulsionskonzentrat in einem Gemisch aus 70 % Lösungsmittel, 20 % Emulgator und 10 % Tensid formuliert.

Zusätzlich enthielten alle Formulierungen oberflächenaktive Zusätze und 11,2 l/ha Ammoniumnitrat-Harnstofflösung (28 % Stickstoff).

Bei diesen Beispielen wurde nach der Methode von S.R. Colby (Weeds 15, 20) derjenige Wert E errechnet, der bei einer nur additiven Wirkung der Einzelwirkstoffe zu erwarten war.

Der Berechnung lag folgende Formel zugrunde:

$$E = X + Y - \frac{XY}{100}$$

Die Variablen der Gleichung haben folgende Bedeutung:

X Prozentuale Wirkung von Wirkstoff A bei einer Konzentration a

Y Prozentuale Wirkung von Wirkstoff B bei einer Konzentration b

E zu erwartende Wirkung (in %) von A + B bei den Aufwandmengen a + b.

Tabelle 1

Synergistische herbizide Wirkung der erfindungsgemäßen Mischung aus dem Triazolderivat 1.002 und dem Herbizid II.005

| Herbizid Nr. II.005 kg/ha | Beispiel Nr. 1.002 kg/ha | Testpflanzen und Schädigung in % (E) | |
|---|---|---|---|
| | | Glycine max. | Amaranthus retrofl. |
| 0.625 | - | 2 (-) | 45 (-) |
| 1.25 | - | 0 (-) | 55 (-) |
| - | 0,125 | 0 (-) | 0 (-) |
| - | 0,25 | 0 (-) | 0 (-) |
| 0,625 | 0,125 | 5 (2)* | 100 (45) |
| 0,625 | 0,25 | 0 (2)* | 98 (45) |
| 1,25 | 0,25 | 8 (0) | 100 (45) |

*) antagonistischer Effekt

Tabelle 2

| Synergistische herbizide Wirkung der erfindungsgemäßen Mischung aus dem Triazolderivat 2.041 und dem Herbizid II.005 | | | |
|---|---|---|---|
| Herbizid Nr. II.005 kg/ha | Beispiel Nr. 2.041 kg/ha | Testpflanzen und Schädigung in % (E) | |
| | | Glycine max. | Amaranthus retrofl. |
| 0,313 | - | 2 (-) | 35 (-) |
| 0,625 | - | 2 (-) | 45 (-) |
| 1,25 | - | 0 (-) | 55 (-) |
| - | 0,125 | 0 (-) | 0 (-) |
| - | 0,25 | 0 (-) | 0 (-) |
| 0,313 | 0,125 | 0 (2)* | 80 (35) |
| 0,313 | 0,25 | 0 (2)* | 98 (35) |
| 0,625 | 0,125 | 0 (2)* | 95 (45) |
| 0,625 | 0,25 | 10 (2) | 95 (45) |
| 1,25 | 0,125 | 8 (0) | 100 (55) |
| 1,25 | 0,25 | 8 (0) | 100 (55) |

*) antagonistischer Effekt

## Ansprüche

1. Triazolverbindungen der allgemeinen Formel I,

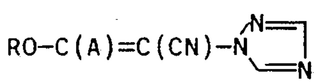

I

in der die Reste folgende Bedeutung haben:

A

$C_1$-$C_{20}$-Alkyl;

Phenyl, Naphthyl oder Pyridyl, wobei diese aromatischen Reste ein bis fünf Halogenatome, einen Phenyl- oder Phenoxyrest und/oder ein bis drei der folgenden Reste tragen können: Hydroxy, Nitro, Amino, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und/oder $C_1$-$C_4$-Halogenalkylthio;

R

Wasserstoff;

eine $C_1$-$C_6$-Alkylgruppe, eine $C_3$-$C_6$-Alkenylgruppe oder eine $C_3$-$C_6$-Alkinylgruppe, wobei diese Gruppen ein bis fünf Halogenatome und/oder einen der folgenden Reste tragen können: $C_1$-$C_4$-Alkoxy, $C_1$-$C_4$-Halogenalkoxy, $C_1$-$C_4$-Alkylthio, $C_1$-$C_4$-Halogenalkylthio, Phenyl;

einen Rest $COR^x$ oder einen Rest $SO_2R^x$, worin

$R^x$

Wasserstoff;

eine $C_1$-$C_8$-Alkylgruppe, welche ein bis drei Halogenatome tragen kann oder Phenyl, welches ein bis fünf Halogenatome und/oder ein bis drei der folgenden Reste tragen kann: Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, oder $C_1$-$C_4$-Halogenalkylthio bedeutet und deren umweltverträglichen Salze.

2. Triazolverbindungen der allgemeinen Formel I gemäß Anspruch 1, in denen A Phenyl bedeutet, welches ein- bis dreifach durch Halogen, $C_1$-$C_4$-Alkyl und/oder $C_1$-$C_2$-Halogenalkyl substituiert sein kann.

3. Herbizide Mittel, enthaltend mindestens eine Triazolverbindung der Formel I gemäß Anspruch 1 oder

eine Triazolverbindung der Formel IA oder IB

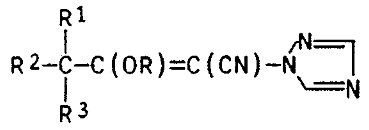

IA

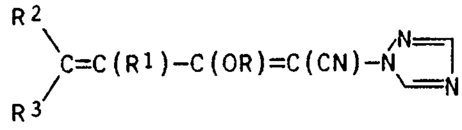

IB

in der die Substituenten folgende Bedeutung haben:
$R^1$
Wasserstoff; eine Cyclopropylgruppe;
eine $C_1$-$C_6$-Alkylgrupe, welche ein bis fünf Halogenatome und/oder ein bis drei der folgenden Gruppen tragen kann: Hydroxy, $C_1$-$C_4$-Alkoxy, $C_2$-$C_4$-Alkenyloxy oder $C_2$-$C_4$-Alkinyloxy;
eine $C_2$-$C_6$-Alkenylgruppe oder eine $C_2$-$C_6$-Alkinylgruppe, wobei diese Gruppen ein bis fünf Halogenatome tragen können;
eine $C_1$-$C_4$-Alkoxygrupe; eine $C_2$-$C_4$-Alkenyloxygruppe oder eine $C_2$-$C_4$-Alkinyloxygruppe;
$R^2$, $R^3$
Wasserstoff; Adamantyl;
eine $C_1$-$C_3$-Alkylgruppe
oder gemeinsam mit dem C-Atom an das sie gebunden sind eine $C_3$-$C_7$-Cycloalkylgruppe, eine $C_6$-$C_{10}$-Bicycloalkylgruppe oder eine $C_6$-$C_{10}$-Bicycloalkenylgruppe, wobei diese cyclischen Reste ein bis drei Halogenatome und/oder $C_1$-$C_3$-Alkylgruppen tragen können und
R
die in Anspruch 1 gegebene Bedeutung hat, oder deren umweltverträgliche Salze sowie mindestens einen herbiziden Wirkstoff aus der Gruppe der Benzothiadiazone der Formel II,

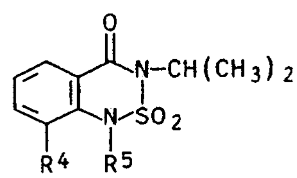

II

in der die Substituenten folgende Bedeutung haben:
$R^4$ Wasserstoff, eine $C_1$-$C_4$-Alkylgruppe, eine $C_1$-$C_4$-Alkoxygruppe oder ein Halogenatom;
$R^5$ Wasserstoff oder Cyano
sowie deren umwelt- und kulturpflanzenverträgliche Salze.

4. Herbizide Mittel gemäß Anspruch 3 enthaltend mindestens eine Triazolverbindung der Formel I, IA oder IB gemäß den Ansprüchen 1 und 2 und mindestens einen herbiziden Wirkstoff der Gruppe der Benzothiadiazone II, wobei das Gewichtsverhältnis von I, IA bzw. IB zu II 10:1 bis 0,01:1 beträgt.

5. Verfahren zur Bekämpfung unerwünschten Pflanzenwuchses, dadurch gekennzeichnet, daß man mindestens ein Triazolderivat I, IA oder IB gemäß Anspruch 3 und ein Benzathiazon II gemäß Anspruch 3 vor, bei oder nach der Aussaat der Kulturpflanzen vor oder während des Auflaufens der unerwünschten Pflanzen gleichzeitig oder nacheinander ausbringt.

6. Verfahren nach Anspruch 5, dadurch gekennzeichnet, daß man die Blätter der unerwünschten Pflanzen gleichzeitig oder nacheinander mit einer synergistisch wirksamen Menge eines Triazolderivates I, IA oder IB und mit einer herbizid wirksamen Menge eines Benzothiadiazinderivates II behandelt.

7. Herbizide Mittel enthaltend mindestens eine Triazolverbindung der Formel I gemäß Anspruch 2 oder deren umweltverträgliche Salze sowie mindestens einen herbiziden Wirkstoff aus der Gruppe der Benzothiadiazone II gemäß Anspruch 3 sowie hierfür übliche inerte Zusatzstoffe.

8. Herbizide Mittel nach Anspruch 6 enthaltend mindestens eine Triazolverbindung der Formel I gemäß Anspruch 2 oder deren umweltverträgliche Salze sowie mindestens einen herbiziden Wirkstoff aus der Gruppe der Benzothiadiazone II gemäß Anspruch 3 wobei das Gewichtsverhältnis von I zu II 10:12 bis 0,01:1 beträgt.

9. Verfahren zur Bekämpfung unerwünschten Pflanzenwuchses, dadurch gekennzeichnet, daß man mindestens eine Triazolverbindung der Formel I gemäß Anspruch 2 oder deren umweltverträgliche Salze sowie mindestens einen herbiziden Wirkstoff aus der Gruppe der Benzothiadiazone II gemäß Anspruch 3, vor, bei

oder nach der Aussaat der Kulturpflanzen vor oder während des Auflaufens der unerwünschten Pflanzen gleichzeitig oder nacheinander ausbringt.

10. Verfahren nach den Ansprüchen 5 und 9, dadurch gekennzeichnet, daß die Kulturpflanzen Gerste, Kultursorghum, Mais, Reis, Weizen, Erbsen, Erdnüsse, Soja und Kulturrasen sind.